# EUROPEAN PATENT APPLICATION

(11) **EP 4 321 101 A1**
(43) Date of publication of application: **14.02.2024**
(21) Application number: 22189986.7
(22) Date of filing: 11.08.2022
(51) Int. Cl.: A61B 6/04, A61B 6/08, A61B 6/00

(54) **PATIENT MOTION DETECTION IN DIAGNOSTIC IMAGING**

(71) Applicant: Koninklijke Philips N.V., 5656 AG Eindhoven (NL)
(72) Inventor: BYSTROV, Daniel, Eindhoven (NL); KROENKE-HILLE, Sven, Eindhoven (NL); SENEGAS, Julien Thomas, 5656AG Eindhoven (NL); JOCKEL, Sascha Andreas, 5656AG Eindhoven (NL)
(74) Representative: Philips Intellectual Property & Standards

(57) **Abstract**

A device (10), system, method and computer-program product are disclosed for detecting movement of a subject in a diagnostic imaging examination. The device comprises a camera/3D surface scanning system (11) and an input (14) for receiving a trigger to indicate that a current spatial configuration of the subject is to be maintained for the examination. The device comprises a processor (12) and an output (13), in which the processor (12) is adapted for acquiring reference data of the subject using the camera/3D system when said trigger is received, and for acquiring further data of the subject using the camera/3D system after acquiring said reference data. The processor is adapted to compare the reference data, which represents a reference state of the subject at substantially the time that the trigger was received, to the further data, which represents a more recent state of the subject. The processor is adapted to provide output data via said output (13), in which this output data is representative of the comparison of the further data to the reference data so as to indicate movement of the subject with respect to the reference state of the subject.

## Description

### FIELD OF THE INVENTION

The present invention relates to the field of diagnostic imaging, and more specifically to a device, system, workstation, method and computer program product for monitoring the positioning (spatial configuration) of a subject (e.g. of one or more body parts of a patient) in a diagnostic imaging environment, e.g. to check for undesirable movement of a patient before commencing a medical imaging procedure, e.g. particularly after initial preparations of the patient to ensure a correct positioning for the examination are completed.

### BACKGROUND OF THE INVENTION

Preparing a patient for an examination by diagnostic imaging, e.g. to diagnose a medical condition and/or plan a treatment, is typically a complex task that heavily relies on manual interventions by trained and experienced personnel. Typically, a skilled operator (e.g. a Medical Technical Assistant, MTA) needs to carefully perform the preparation and setup for a session in accordance with an established protocol and/or specific guidelines regarding the correct positioning of the patient and the configuration of the image acquisition, e.g. configuration of the diagnostic imaging system.

For example, for the acquisition of an X-ray image, a patient needs to be properly positioned with respect to the X-ray imaging system (and/or vice versa). This typically implies that the patient needs to take a certain pose in relation to the image detector and the X-ray tube. For example, limbs and joints may need to be positioned and/or oriented precisely in relation to the detector and tube as indicated by the diagnostic purpose of the examination. After this positioning, the patient is typically instructed to avoid any further movement, but the X-ray image will generally not be acquired immediately after the positioning. For example, the operator may need to configure the system parameters for the acquisition and/or to retreat to a safer distance and/or behind a radiation shield (or to a different room) to avoid exposure of the operator to potentially damaging radiation (which, even though typically a very small dose, could still accumulate over time in typical, e.g. daily, practice without suitable protective measures).

However, if the patient moves after the correct pose has been assumed, e.g. as aided and confirmed by the operator, but before the image acquisition is performed, the X-ray image(s) may be unsuitable or suboptimal for the intended diagnostic purposes. Therefore, a retake of the image might be necessary in such case. Even though digital radiography techniques, fortunately, allow to quickly detect whether the diagnostic quality of the acquired image is sufficient, e.g. within minutes, if not merely seconds, it will be understood that repeating an image acquisition has several undesirable consequences: the patient is exposed to an additional radiation dose that could have been avoided, and time and other resources, such as due to the power drawn by the system in use and/or wear and tear of the system (e.g. of the X-ray tube), would also be unnecessarily wasted.

Various approaches for improving the patient positioning process that rely on optical (digital) camera imaging are known in the art. For example, it is known to display a target location, or more generally, target spatial configuration, of a limb or body part to the patient, in combination with live camera imaging feedback, so as to guide the patient to the correct position, see e.g. US 2015/003674 A1 and US 2017/020469 A1.

Thus, visual feedback may be provided to bring the patient into a desired position for the diagnostic imaging examination. However, such techniques may typically rely on specific processing to determine a correct (desirable) position for the examination, e.g. in a reference frame tied to the diagnostic imaging system and/or the optical camera (the correspondence between these coordinate systems being typically precisely established), to detect the current spatial configuration of the relevant body part(s) of the patient, and/or to compare the current position to the desired state. This may involve specific, possibly intricate, processing techniques, which can increase the costs of the development, production and/or maintenance of the system, could limit the flexibility of the system (e.g. only supporting common examinations, typical body shapes and/or standard use cases), might create risks in view of incorrect inference of information from the observed images, e.g. detection of a body part or parts, and/or might incorrectly determine the recommended spatial configuration for an atypical patient (e.g. in view of injuries, congenital disorders and/or other medical conditions).

Even though various methods are known in the art, including the examples hereinabove, to assist in the process of guiding a patient into a certain position that is required (or desirable) for a specific imaging examination, a need exists in the art to provide a simple and robust approach to monitor the positioning of the patient, preferably without using (or, at least, relying on minimal) complicated image recognition and/or processing techniques, and preferably also without relying on predefined definitions of and/or assumptions about the desired geometrical state (i.e. the "position" in its broadest sense), e.g. in an examination-agnostic and patient-agnostic manner, i.e. irrespective of the specific (type of) examination and/or patient.

When the operator has finished positioning the patient, other actions may be required before commencing the acquisition process. For example, the X-ray device may be configured and/or fine-tined for the examination, e.g. the collimation of the X-ray beam and/or other settings may need to be adjusted. After the patient setup and any configuration steps that need to be performed locally at the imaging site (e.g. where the X-ray tube and/or detector are located; it is to be noted that the remotely vs. locally controllable settings may vary from system to system), the operator typically leaves the immediate vicinity of the patient (e.g. leave the imaging site, e.g. in an acquisition room) to control the image acquisition process from a console (e.g. in a control room). Therefore, the actual X-ray image acquisition could take place after a non-negligible period of time has passed after the positioning setup. Furthermore, the operator may not be able to observe the patient directly or in sufficient detail during the final steps before the acquisition is executed. Embodiments of the present invention can address the need for a simple technique to ensure that the patient does not move after the preparation step (the positioning of the patient), i.e. such that the patient essentially freezes until the (e.g. X-ray) diagnostic image(s) are acquired.

### SUMMARY OF THE INVENTION

It is an object of embodiments of the present invention to provide in simple, good and/or efficient means and methods to monitor the spatial configuration (the "position," in a general sense) of a subject (e.g. of a patient, e.g. of at least one body part or parts of the patient that is specifically of interest for the imaging examination) in a diagnostic imaging environment, e.g. to check for undesirable movement of a patient before commencing a medical imaging procedure, such as an X-ray image acquisition. Particularly, embodiments may be used to ensure that a correct positioning of the subject for an imaging examination is maintained after the initial preparations to setup the patient have been performed, e.g. before the actual diagnostic image acquisition is executed.

It is an advantage of embodiments of the present invention that poor diagnostic image quality (e.g. insufficient for diagnostic purposes) and/or a retake of a diagnostic image due to movement of the patient after the positioning and before the acquisition can be avoided or reduced. It is also an advantage that an additional radiation dose exposure of the patient due to a retake of the diagnostic image can be avoided or (e.g. at least statistically) reduced. Likewise, it is also an advantage that radiation exposure of sensitive organs or tissues, positioned to be outside the collimated radiation field (e.g. being not of interest for the intended examination) but nonetheless exposed due to undesirable movement of the patient can be reduced or avoided. Wasted resources, e.g. of time, electricity, wear and tear of the system and the like, due to unnecessary retakes of diagnostic images can also be reduced or avoided.

Furthermore, if a suboptimal positioning of the patient at the time of the diagnostic image acquisition is not detected in time by the operator, a risk of an incorrect or inconclusive diagnosis might arise, which can also be reduced by embodiments of the present invention. It will be understood that the costs associated with inviting the patient to return to the facility for repeating the examination at a later time (e.g. date) in view of poor diagnostic image quality that went unnoticed at the time of the earlier acquisition are even higher than when an immediate retake of the image would be performed.

It is an advantage of embodiments of the present invention that a camera system (e.g. one or more cameras) can be used that do not need to be specifically and/or accurately aligned with the diagnostic imaging system, e.g. an accurate determination of a correspondence between the camera coordinate system(s) and the diagnostic imaging coordinate system, such as by a calibration procedure, is not required. For example, in a system that automatically determines the desired spatial configuration of the patient and/or guides the patient (possibly via interaction with the operator) to this desired configuration, an accurate mapping is typically needed to relate camera observations to the coordinate frame of the diagnostic image(s) intended to be taken. However, embodiments of the present invention may monitor the patient for signs of (any substantial) movement after a trigger is received to indicate that the desired position has been achieved, such that knowledge of the coordinate system of the diagnostic imaging system (and thus of the diagnostic image to be acquired) is not needed. This may lead to an approach that is very robust (e.g. errors in coordinate mapping are avoided, and/or changes in the camera alignment overtime are not necessarily problematic) and simple to install and use (e.g. no detailed calibration procedure is needed). Furthermore, the camera system does not need to be integrated into the diagnostic imaging system, e.g. may be installed as a simple addon without requiring a complex integration, or even any substantial co-integration, into the diagnostic imaging system (e.g. data connections, precise mechanical linkages, etc.).

It is an advantage of embodiments of the present invention that a simple and robust approach is applied to detect movement of the patient (or of his/her relevant body part or parts) that does not rely on specific, e.g. complex, processing techniques to determine the correct (or, at least, a desirable) spatial configuration of the patient's body for a specific examination, to detect the current spatial configuration of the patient's body or its relevant body part(s) (e.g. by fitting a model of the body or parts thereof to live camera images), and/or to compare the current state to the desired state. By avoiding such intricate processing techniques, the costs of development, production and/or maintenance of a system in accordance with embodiments may be kept low, and a high degree of flexibility can be provided, e.g. the system does not rely (extensively) on predefined desirable (i.e. reference) states for specific examinations (e.g. embodiments can be applied to essentially any examination without need for prior knowledge), nor on assumptions regarding body shape, condition and/or other characteristics of the specific patient. It will be understood that a simple approach that minimizes assumptions and/or the use of (machine-learned and/or codified) prior knowledge may avoid or, at least, reduce risks due to incorrect estimation and/or inference of information (e.g. detection of body features, model fitting, ...). The flexibility thus provided advantageously allows the operator to use his/her experience and best judgement for positioning the patient, without being constrained by a reference condition (patient position/spatial configuration) defined and/or determined by the system.

However, it is noted that some (e.g. limited and/or simple) image processing is not necessarily excluded. For example, landmark features, which may (preferably) be easily and robustly computable, may be detected in the camera image(s). The use of landmark features may allow a more reproducible quantification and/or characterization of movement of the patient, e.g. the check of a quantified degree of movement against a predetermined threshold of 'tolerable' motion, without requiring detailed knowledge of the imaging procedure to execute or of specific characteristics of the patient, such that the approach in accordance with embodiments can still be considered to be advantageously procedure-agnostic and patient-agnostic.

It is an advantage of embodiments of the present invention that an arbitrary position (i.e. spatial configuration) of the patient (or of his/her relevant body part or parts) can be used as reference to detect motion, e.g. a substantial deviation of the patient's position from this reference, in a simple manner, e.g. without requiring programming, (machine) training and/or explicit definition of the reference.

It is an advantage of embodiments of the present invention that position information of the patient, and/or of specific body part(s), and/or movement information (e.g. changes in this position information) can be conveniently monitored by the operator, e.g. using (an) overlay(s) shown on a display to the operator. Additionally or alternatively, such information may be displayed to the subject, e.g. so as to allow the subject to recognize a change in his/her position and to correct such motion.

It is an advantage of embodiments of the present invention that depth and/or 3D camera imaging may be used to allow a change of spatial configuration of the patient to be detected in substantially three dimensions, e.g. changes in position (and, by extension, orientation, etc.) that occur in the direction perpendicular to the camera imaging plane (or, at least, that have a substantial component in this depth direction) can be detected in addition to the in-plane changes that would also be detectable by using a conventional 2D camera.

Depth (e.g. 3D) imaging may also, advantageously, allow a good detection of movement by increasing the separability and detectability of the patient (or, generally, his/her relevant body part or parts) in the camera (depth/3D) image(s), such that changes in the spatial configuration can be more easily detected. Even if the body part(s) has (have) a poor image contrast to the background, it may typically be positioned at some distance from its background, as viewed from the camera's perspective, such that it can be easily detected and dynamically compared (to detect changes in position, orientation, ..) to a reference image (e.g. captured earlier upon receiving a trigger to register the patient's position as the desired state for the diagnostic image acquisition). It is noted that this does not require any assumptions regarding the shape or other properties of the body part(s), only that it is sufficiently physically removed from surrounding object(s). Even if the body part is placed in contact with a surface, e.g. a plane of the detector or detector enclosure (and the camera observes from a generally opposite direction, e.g. from more or less the vantage point of an X-ray tube, without any limitation thereto), it will be understood that the thickness of the (and, generally, any arbitrary) body part will make it, figuratively and literally, stand out in an acquired depth or 3D image. This also reduces false detection of movement due to, for example, moving shadows, changes is lighting and/or other such observable changes in a mere 2D (e.g. color or grayscale) image that are not related to actual changes in position of the actual object (the body part or parts).

Furthermore, it is also an advantage of embodiments of the present invention that an objective assessment value can be determined to indicate whether it is necessary, or would be advisable, to reposition the subject (e.g. return to the patient positioning preparation phase) in order to obtain good diagnostic image quality, e.g. an X-ray image with sufficiently high quality for diagnostic purposes. Such assessment may be made by the operator (e.g. a MTA), but may also be determined automatically in accordance with embodiments of the present invention (e.g. as an automatically generated advice to the operator). When the assessment, as determined by the system or method in accordance with embodiments, is negative, the execution of the prepared imaging examination may even be blocked, e.g. disabling a button or other signal source to activate the acquisition process, such that the operator is not only alerted, but also forced to take corrective action before the acquisition can take place (it will be understood that, in such case, an override option may be provided to allow the operator to continue anyway, relying on his/her experience and professional judgement).

Even though the automatic generation of an assessment (i.e. a value indicating whether observed changes in the patient's positioning are likely to reduce the diagnostic image quality substantially) may (e.g. optionally) take some (e.g. limited) prior knowledge of the imaging procedure to be performed explicitly or implicitly into account, it will be understood that this is different from, starting from a predefined procedure (e.g. using a geometric 3D model of the patient and/or otherwise codified definition of the preferred patient positioning for a specific procedure), automatically guiding the patient to the position prescribed for this procedure and comparing the patient's position to the reference, e.g. based on a model. Embodiments of the present invention allow the operator to position the patient in any way he/she deems suitable for the intended purpose, free of any constraints imposed by an automated patient positioning guidance system. After the operator has indicated that the patient is positioned correctly, changes in position can be detected in an automated way, and, for example, when the detected movement exceeds a predetermined threshold, the operator (and/or the patient) may be alerted. The reference position, e.g. a snapshot image of the patient taken at the moment the operator has indicated that the positioning preparation is concluded, and the currently observed position (e.g. live camera images), or information derived therefrom, may be processed to determine said assessment value. For example, a machine learning algorithm (e.g. a trained artificial intelligence model) may use this information to estimate whether a diagnostic image taken at that time, i.e. with the patient positioned as observed vs. as originally intended by the operator (e.g. the reference image), would (likely) be of sufficient quality. However, this does not limit the flexibility or usability of the system and/or method in accordance with embodiments: the operator remains free to position the patient in any way deemed suitable in his/her best judgment when defining the reference state (e.g. a reference snapshot).

It is an advantage of embodiments of the present invention that a partially automated, e.g. machine-assisted, procedure is provided to routinely improve the workflow and/or the efficiency of patient positioning tasks in diagnostic imaging examinations, e.g. X-ray examinations, e.g. projection X-ray radiography examinations.

It is an advantage of embodiments of the present invention that embodiments of the present invention can provide real-time, e.g. substantially continuously updated (or, at least, updated at a reasonably high frequency), monitoring of the patient's spatial configuration (e.g. position) so as to detect motion (e.g. exceeding a predetermined threshold) away from an earlier spatial configuration of the patient that was selected as reference.

It is an advantage of embodiments of the present invention that the motion detection approach (or, in other words, change detection for a spatial configuration of the subject), as provided by embodiments, can be applied, without (or, with little) modification, registration, mathematical modeling and/or initial configuration, in combination with any diagnostic imaging system, e.g. any generic, non-standard, third-party, custom-made and/or, generally, arbitrary imaging system. In other words, the approach does not rely on knowledge of characteristics, parameters, configuration, features and/or type of the diagnostic imaging system, nor on knowledge of the diagnostic imaging procedure that will be performed, nor on knowledge of characteristics of the subject. However, it will be understood that, if deemed useful, some (e.g. limited) prior knowledge about the system, examination and/or subject may be used in accordance with some embodiments of the present invention (i.e. such embodiments are not necessarily excluded).

A method, computer program product, device, system and/or workstation in accordance with embodiments of the present invention achieves the above objective.

In a first aspect, the present invention relates to a method, e.g. a computer-implemented method, to detect movement of at least one body part of a subject in a diagnostic imaging examination, e.g. an X-ray imaging examination, e.g. an X-ray projection radiography examination. The method comprises receiving a trigger signal to indicate that a current spatial configuration of the subject is to be maintained for the diagnostic imaging examination. The method comprises, when said trigger signal is received, acquiring reference image and/or spatial data of the subject using a camera and/or 3D surface scanning system. The method further comprises, after said reference image and/or spatial data of the subject is acquired using the camera and/or 3D surface scanning system, acquiring a further image and/or spatial data of the subject using the camera and/or 3D surface scanning system. The method also comprises comparing the reference image and/or spatial data of the subject, which represents the subject's state at substantially the time that the trigger was received, to the further image and/or spatial data of the subject, which represents a more recent state of the subject (or vice versa, the further data to the reference data). The method comprises providing an output to the operator and/or to the subject that is representative of said comparison of the further image and/or spatial data to the reference image and/or spatial data to indicate movement of the subject with respect to the reference state of the subject.

In a method in accordance with embodiments of the present invention, the output may be provided via at least one display monitor and/or human interface device.

In a method in accordance with embodiments of the present invention, the output may be provided as an image overlay, displaying said comparison in the form of a difference image as an overlay over said further image and/or spatial data.

In a method in accordance with embodiments of the present invention, the further image and/or spatial data of the subject may be acquired repeatedly, periodically and/or substantially continuously so as to obtain a live stream of further image and/or spatial data, wherein said reference image and/or spatial data is compared repeatedly to the most recently acquired further image and/or spatial data of said live stream, and said output is repeatedly provided to present a dynamic view of the current change of spatial configuration of the subject with respect to the earlier state of the subject.

In a method in accordance with embodiments of the present invention, the trigger signal may be received from an operator via a human interaction interface, e.g. a button, a voice control interface and/or a gesture detection system to detect a gesture made by the operator.

A method in accordance with embodiments of the present invention may comprise detecting proximity of the operator to the subject using radiofrequency identification tag detection, an indoor positioning system, a light beam gate, a sonar, radar and/or lidar system, and/or another sensor system for presence, proximity and/or position detection, wherein said trigger signal is generated when said proximity detection indicates that the operator has left the vicinity of the subject.

In a method in accordance with embodiments of the present invention, the trigger signal may be received from an automated system for detecting a predetermined reference spatial configuration of the subject, as required for the diagnostic imaging examination, in a live imaging stream from the camera and/or 3D surface scanning system.

A method in accordance with embodiments of the present invention may comprise acquiring a live imaging stream from the camera and/or 3D surface scanning system, performing a scene analysis of the live imaging stream to detect a predetermined condition, and generating said trigger in response, in which the predetermined condition corresponds to the operator leaving the immediate vicinity of the subject.

In a method in accordance with embodiments of the present invention, the reference image and/or spatial data may be acquired at the moment of receiving the trigger or a predetermined short time thereafter, and/or the reference image and/or spatial data may be selected from a buffer storing a stream of image and/or spatial data acquired from the camera and/or 3D surface scanning system, in which the selection corresponds to a recent point in time before the trigger was received.

In a method in accordance with embodiments of the present invention, acquiring the reference image and/or spatial data of the subject using the camera and/or 3D surface scanning system may comprise acquiring a conventional monochrome and/or color digital photographic image, in the visible and/or infrared spectrum.

In a method in accordance with embodiments of the present invention, acquiring the reference image and/or spatial data of the subject using the camera and/or 3D surface scanning system may comprise acquiring the reference image and/or spatial data using a plurality of cameras, comprised in the camera system, concomitantly, so as to obtain image and/or spatial information of the subject from different vantage points.

In a method in accordance with embodiments of the present invention, acquiring the reference image and/or spatial data of the subject using the camera and/or 3D surface scanning system may comprise acquiring 3D and/or depth information.

In a method in accordance with embodiments of the present invention, comparing the reference image and/or spatial data of the subject may comprise detecting at least one image feature and/or landmark in the reference image and/or spatial data as well as in the further image and/or spatial data, and comparing a position of the at least one image feature and/or landmark between the reference and further image and/or spatial data for use in determining said output.

In a method in accordance with embodiments of the present invention, the image feature and/or landmark may comprise at least one anatomical landmark on a joint, bone, muscle and/or other externally discernable anatomical feature of the subject's body.

In a method in accordance with embodiments of the present invention, providing the output may comprise determining a difference measure indicative of movement of the subject relative to the reference state based on said comparison, and, when said difference measure exceeds a predetermined threshold, alerting the operator that the subject is no longer in the intended reference position.

In a second aspect, the present invention relates to a device for detecting movement of at least one body part of a subject in a diagnostic imaging examination, e.g. an diagnostic X-ray imaging examination, e.g. a projection X-ray radiography examination. The device comprises a camera and/or 3D surface scanning system for acquiring image and/or spatial data of the subject and an input for receiving a trigger signal to indicate that a current spatial configuration of the subject is to be maintained for the diagnostic imaging examination. The device comprises a processor and an output, in which the processor is adapted for acquiring reference image and/or spatial data of the subject using the camera and/or 3D surface scanning system when said trigger signal is received, and for acquiring further image and/or spatial data of the subject using the camera and/or 3D surface scanning system after said reference image and/or spatial data of the subject is acquired. The processor is furthermore adapted to compare the reference image and/or spatial data of the subject, which represents a reference state of the subject at substantially the time that the trigger signal was received, to the further image and/or spatial data of the subject, which represents a more recent state of the subject (or, equivalently, comparing said further data to said reference data, i.e. vice versa). The processor is adapted to provide output data to the operator and/or to the subject via said output, wherein said output data is representative of said comparison of the further image and/or spatial data to the reference image and/or spatial data so as to indicate movement of the subject with respect to the reference state of the subject.

In a device in accordance with embodiments of the present invention, the processor may be adapted to repeat the steps of acquiring the further image and/or spatial data via the camera and/or 3D surface scanning system, comparing the reference image and/or spatial data to the further image and/or spatial data, and providing the updated output data to present a dynamic view of a current change of the spatial configuration of the subject with respect to the earlier reference state of the subject.

In a device in accordance with embodiments of the present invention, the output may comprise a display monitor. The processor may be adapted for presenting, via the display monitor, a visual representation of the comparison.

In a device in accordance with embodiments of the present invention, the processor may be adapted to present, in said output data, the shape, area and/or volume of the two-dimensional and/or three-dimensional difference determined by said comparison as an overlay and/or a contour on a visualization of the further image and/or spatial data and/or as a marker and/or annotation accompanying such visualization.

In a device in accordance with embodiments of the present invention, the processor may be adapted to determine a difference measure indicative of movement of the subject relative to the reference state based on said comparison, and outputting, via said output, an alert to the operator and/or the subject and/or a signal to the diagnostic imaging system when said difference measure exceeds a predetermined threshold, so as to indicate that the subject is no longer in the intended reference state.

In a device in accordance with embodiments of the present invention, the camera and/or 3D surface scanning system may comprise at least one camera and/or 3D surface scanning device arranged so as to obtain a two-dimensional and/or three-dimensional view of the at least one body part and/or of the subject, wherein said at least one camera and/or 3D surface scanning device comprises an optical camera for acquiring a monochrome, color and/or multispectral two-dimensional image, in the infrared and/or visible spectrum, and/or a plurality of such cameras set up so as to view the subject from different angles, and/or a depth camera and/or a 3D surface imaging system.

In a device in accordance with embodiments of the present invention, the input may comprise a human interaction interface.

In a device in accordance with embodiments of the present invention, the input, e.g. the human interaction interface, may comprise a button, a voice control interface and/or a gesture detection system to detect a gesture made by the operator.

In a device in accordance with embodiments of the present invention, the input may comprise a proximity and/or position detection system to detect proximity of the operator to the subject, so as to generate said trigger signal when the operator leaves the vicinity of the subject.

In a device in accordance with embodiments of the present invention, the input, e.g. the proximity and/or position detection system, may comprise a radiofrequency identification tag in combination with at least one radiofrequency identification tag detection sensor, and/or may comprise at least one light gate, and/or may comprise a sonar, radar and/or lidar system, and/or may be implemented by detecting, using the processor, the position of the operator in a live stream of image and/or spatial data of the subject acquired by the camera and/or 3D surface scanning system.

In a device in accordance with embodiments of the present invention, the input may comprise a connection for receiving the trigger signal from an automated system for detecting a predetermined reference spatial configuration of the subject, as indicated for the diagnostic imaging examination, in a live imaging stream provided by the camera and/or 3D surface scanning system.

A device in accordance with embodiments of the present invention may comprise said automated system for detecting the predetermined reference spatial configuration of the subject.

A device in accordance with embodiments may comprise an artificial intelligence module for evaluating a trained machine learning model and to generate said trigger signal (provided to the input) by taking an output of the evaluated model into account. The trained machine learning model uses, as input, said image and/or spatial data, and/or data from the diagnostic imaging system, and/or data derived therefrom. The data from the diagnostic imaging system (to be received as input in operation of the device) may comprise device state information of the diagnostic imaging system and/or control input information received by the diagnostic imaging system from user interactions.

In a device in accordance with embodiments of the present invention, the processor may be adapted for comparing the further image and/or spatial data with the reference image and/or spatial data by detecting at least one image feature and/or landmark in the reference image and/or spatial data and the corresponding at least one image feature and/or landmark in the further image and/or spatial data, and comparing a position of the at least one image feature and/or landmark between the reference image and/or spatial data and the further image and/or spatial data.

In a third aspect, the present invention relates to a diagnostic imaging system, in which the system is adapted to perform a method in accordance with embodiments of the first aspect of the present invention and/or comprises a device in accordance with embodiments of the second aspect of the present invention.

The dagnostic imaging system in accordance with embodiments of the present invention may comprise an X-ray imaging system, e.g. a projection X-ray imaging system.

In a fourth aspect, the present invention relates to a workstation for a diagnostic imaging system, e.g. a diagnostic X-ray imaging system, in which the workstation is adapted to perform a method in accordance with embodiments of the first aspect of the present invention and/or comprises a device in accordance with embodiments of the second aspect of the present invention.

In a fifth aspect, the present invention relates to a computer-program product for performing, when executed on a computer (e.g. a device in accordance with embodiments of the second aspect of the present invention), a method in accordance with the first aspect of the present invention.

The independent and dependent claims describe specific and preferred features of the invention. Features of the dependent claims can be combined with features of the independent claims and with features of other dependent claims as deemed appropriate, and not necessarily only as explicitly stated in the claims.

### BRIEF DESCRIPTION OF THE DRAWINGS

Fig. 1 shows a method in accordance with embodiments of the present invention.
Fig. 2 shows an illustrative visualization (e.g. comprised in an output of a method and/or device in accordance with embodiments) of patient movement with respect to a frozen reference image, to illustrate embodiments of the present invention.
Fig. 3 shows, schematically, a device in accordance with embodiments of the present invention.
Fig. 4 shows a diagnostic imaging system in accordance with embodiments of the present invention, and a workstation for a diagnostic imaging system in accordance with embodiments of the present invention.

The drawings are schematic and not limiting. Elements in the drawings are not necessarily represented on scale. The present invention is not necessarily limited to the specific embodiments of the present invention as shown in the drawings.

### DETAILED DESCRIPTION OF EMBODIMENTS

Notwithstanding the exemplary embodiments described hereinbelow, is the present invention only limited by the attached claims. The attached claims are hereby explicitly incorporated in this detailed description, in which each claim, and each combination of claims as allowed for by the dependency structure defined by the claims, forms a separate embodiment of the present invention.

The word "comprise," as used in the claims, is not limited to the features, elements or steps as described thereafter, and does not exclude additional features, elements or steps. This therefore specifies the presence of the mentioned features without excluding a further presence or addition of one or more features.

In this detailed description, various specific details are presented. Embodiments of the present invention can be carried out without these specific details. Furthermore, well-known features, elements and/or steps are not necessarily described in detail for the sake of clarity and conciseness of the present disclosure.

In a first aspect, the present invention relates to a method to check for movement of a subject (e.g. of at least one body part of the subject) in a diagnostic imaging environment, e.g. to check for undesirable movement of a patient before commencing a medical imaging procedure, e.g. particularly after initial preparations to bring the subject in a correct position (pose, or, generally, a desired spatial configuration) for the examination are completed. Movement may thus be detected with respect to an earlier position and spatial configuration of the subject before performing the diagnostic image acquisition. A position, orientation, deformation and/or other spatial (geometrical) properties of the subject may be monitored to detect a change thereof, such that the spatial relationship between the subject and the diagnostic imaging system (e.g. a detector and a X-ray tube) remains substantially constant.

It will be understood that principles of the present invention may equally apply to various types of diagnostic imaging examination, and are not necessarily limited to X-ray imaging, in general, or to X-ray projection radiography, specifically. References made throughout the present disclosure to X-ray imaging and the like are thus merely intended for illustrative purposes, and are not necessarily limitative.

However, it will also be understood that embodiments of the present invention might be particularly advantageous and/or useful in view of the specific circumstances of X-ray imaging and/or X-ray projection imaging. For example, the configuration process for an X-ray examination, e.g. of beam collimation, tube voltage (kVp), tube current, filtration parameters and/or other settings, and the need for radioprotective precautions for the sake of safety of the operator may typically lead to a substantial time span in between the moment that the patient has assumed the desirable position for the examination and the actual moment of acquisition, during which the operator may not be able to (easily) verify that the patient has maintained the desired position.

Moreover, conventional (e.g. relatively simple) projection radiography systems may often lack many automation features and/or support systems that allow a high degree of remote control of the system from a control room (e.g. remote collimation, automatic insertion/configuration of beam filtration plates, an automated patient couch, etc.) and/or may lack means for high-quality remote patient observation (e.g. by high resolution cameras and/or cameras for simultaneous observation of the patient from different observation angles), such that the time during which the patient is expected to remain still in the intended position while the operator is distracted by other (often manual) tasks and/or not able to easily confirm visually that the patient's position has not changed may be particularly problematic in conventional projection radiography examinations using such system.

The method in accordance with embodiments of the present invention may be a computer-implemented method, i.e. an automated or semi-automated method that can be performed by dedicated processing hardware (e.g. using an application specific integrated circuit) and/or configured/programmed processing hardware, such as a computer or other general-purpose processor that is programmed for the specific task of performing the method (e.g. by executing a specific software designed for said purpose) and/or a configurable hardware platform (e.g. a field programmable gate array) configured for the specific task of performing the method. Combinations of application-specific hardware (e.g. ASIC) and/or configured hardware (e.g. FPGA) and/or one or more programmed processing devices (e.g. using a CPU, GPU or other suitable processor, typically in combination with supporting hardware, e.g. as commonly found in computers) are also possible. The method may also be performed by a plurality of processors, computers and/or other processing devices acting collaboratively, e.g. using a host-client architecture, web-based architecture, cluster processing, and/or other form of distributed processing, data collection, data presentation and/or data storage.

"Automated" and "semi-automated" may refer to a computer-implemented method implemented by a (e.g. digital) processor, controller and/or other such hardware, in which the method is performed in an autonomous or supervised autonomous mode, e.g. requiring only limited input from and/or interaction with an operator, e.g. to select or enter relevant parameters and/or configuration options, to start, stop and/or interrupt the procedure, to supervise the procedure and/or other such limited interactions.

Where reference is made to "camera," "depth camera," "3D camera" or similar components, it will be understood that the device to which is referred is not to be confused with the diagnostic imaging system as such (or a diagnostic imaging component thereof). Even though the "camera" may be implemented as a part of the imaging system, by default or by suitable modification, the primary purpose of the diagnostic imaging system will be understood to relate typically to an image acquisition process by different means, e.g. by ionizing radiation, radiofrequency signals, ..., and is not essentially (or at least not solely) based on the "camera" observation as such. For example, even though the radiation detection component (i.e. image detector) of a PET or SPECT system can be referred to as a PET or SPECT "camera," it will be understood that this differs from the "camera" in the sense of the present disclosure.

Cameras in the sense of the present disclosure may for example include conventional optical imaging cameras and/or depth imaging cameras. The former (conventional cameras) may generally relate to imaging cameras that detect (i.e. particularly in order to determine an image from) light in the (human) visible spectrum and/or infrared spectrum. The conventional camera may for example be adapted for monochrome, color and/or multispectral imaging. The latter (depth cameras) generally relates to technologies to detect depth information in addition to in-plane information (e.g. the two-dimensional projection plane of an image detector), e.g. using stereo imaging (or, more generally, camera imaging from multiple different viewpoints simultaneously), range cameras, LIDAR, RADAR, and/or other such techniques to determine points and/or surfaces in three-dimensional space, e.g. such that a surface contour (e.g. facing the camera) of (an) object(s) can be qualified at least to some extent in three dimensions. In this context, a depth camera is understood to refer generally to any suitable means for gathering spatial data characterizing the observed scene in three spatial dimensions, e.g. such that the (3D) position in space of points on surfaces (at least when not obscured from the camera viewpoint) of objects in the observed scene are determined. It is noted that, where reference is made to the camera system hereinbelow, this may equally refer to a 2D image camera, a depth or 3D camera, or a 3D surface scanning system (or any combination thereof).

Some examples, without limitation thereto, of optical and/or other range sensing techniques that may be suitable for use in the camera and/or 3D surface scanning system in accordance with embodiments include a structured light system, a stereo vision system and/or an active-stereo system. An active-stereo system refers to a stereo vision system that uses, additionally, structured light to improve the detection of surface details, e.g. in which a structured light pattern (e.g. using infrared light) is used to actively provide optically detectable detail to homogeneous surface regions. Range measurements may be acquired at a single point, across a scanning plane and/or over an entire volume in space, e.g. a full image with depth measurements at every point in the sensors field-of-view.

The method in accordance with embodiments of the present invention may be used to assist in patient positioning during an imaging session, e.g. in a preparation step of a projection X-ray imaging examination, particularly after positioning the patient (e.g. aided by an operator) and before executing the diagnostic image acquisition(s). Where reference is made to a diagnostic (or medical) imaging session or environment, it will be understood that this may refer to a procedure (resp. environment therefor, e.g. an examination room) in which the (e.g. human) subject is positioned in a desired pose, or generally desired spatial configuration, with respect to the imaging system (e.g. with respect to the X-ray tube and X-ray detector) to perform the imaging procedure, after which one or a plurality of diagnostic images may be acquired. Embodiments of the present invention may be particularly useful in cases where the patient is supposed to remain still in the desired pose until completion of the acquisitions. This can be particularly problematic where the patient is not aided by a support or specific positioning means, e.g. in a routine examination in which the patient is standing (e.g. upright) or sitting (e.g. on a stool) without much mechanical support of the body, yet has to remain still in the desired orientation and/or position with respect to a detector plane. This may e.g. be often the case for projection radiography examinations to diagnose bone fractures and the like (without limitation thereto).

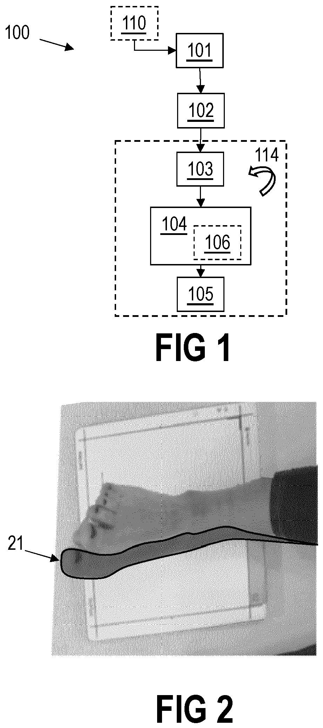

Referring to Fig. 1, an illustrative method 100 in accordance with embodiments of the present invention is shown. The method, e.g. computer-implemented method, 100 can be used to assist in the positioning of a subject, e.g. to avoid movement from a desired position/pose (i.e. spatial configuration), e.g. during and/or after a preparation step of the diagnostic imaging session.

The method 100 comprises receiving 101 a trigger signal to indicate that a current spatial configuration of the subject is to be maintained.

This trigger may be provided, manually, by the operator. For example, an operator (e.g. a medical technical assistant, MTA) may bring the patient into a position (generally, a spatial configuration) that is deemed suitable or desirable, e.g. optimal, for the intended diagnostic imaging examination. The operator can exercise his/her own best judgment, relying on own experience and knowledge, e.g. preferably without being constrained by any substantial limitations imposed by the method or system in accordance with embodiments. Once the subject is in the desired position, the operator provides the trigger to indicate that this position (i.e. spatial configuration) of the subject is to be maintained.

However, alternatively or additionally (e.g. using an "OR" logic connection), the method (e.g. a system integrating the method) may also be combined with a different method to determine and match the correct positioning of the patient automatically, e.g. a machine vision algorithm to detect when the patient is in a predetermined position (spatial configuration) suitable for the examination. While such algorithms are known and generally outside the scope of the present application, The trigger may thus be generated by a further system to assist in the positioning of the patient. As illustrated by the examples in the background section hereinabove, various approaches are known in the art to guide a patient into a pose suitable for an examination, and therefore not discussed in detail. The method in accordance with embodiments may also comprise such prior-art method to assist in positioning the subject, e.g. performed prior to the step of receiving 101 the trigger. The trigger may be generated, as intermediate output, by this (automated or semi-automated) positioning assistance step, or the positioning assistance step may be performed prior to the operator performing a manual confirmation of the patient's position and generating the trigger manually. In other words, the method 100 may comprise a step of positioning assistance to bring the patient into a predetermined, or automatically determined, spatial configuration. The trigger may thus be (e.g. automatically) generated by (a system implementing) the step of positioning assistance, or may be received as an external input (e.g. due to an action performed by a user, i.e. the operator).

The trigger may be generated by a human interaction interface, such as by the press of (or more generally, the use of) a button or switch, by a voice interface, by a gesture detection (e.g. detecting a gesture of the operator), by a keyboard and/or mouse interaction with a (e.g. graphical) user interface, and/or by any other suitable means for interacting with a user, i.e. for receiving a signal from the operator (e.g. at least a one-bit signal in any suitable form). Thus, a hardware button (possibly included in a general purpose interface, such as a keyboard and/or mouse), a gesture detection or voice control system may be used to indicate the moment in time of good positioning of the subject for the examination.

For example, the camera system (discussed further in detail hereinbelow) may be used to detect a gesture from the operator. Even though the operator is preferably not visible in the reference image and/or further spatial data (discussed hereinbelow), the operator may transiently provide a (machine-) detectable gesture, such as a swiping motion over the camera view (e.g. moving the hand over the camera lens from a short distance), or a more complex gesture, such as a thumbs-up gesture. For example, moving a hand over the camera, or, generally, obscuring a substantial part of the camera's field-of-view by the hand (or, equivalently, by an alternative body part or object), e.g. in a swiping, grabbing or other gesture, motion or hand posture at short distance, may be particularly easy to detect with good sensitivity, accuracy and robustness. Thus, the trigger signal can be easily generated, in accordance with an embodiment of the invention, by detecting a large change in the camera image, such as a substantial drop of the average image intensity of a conventional optical camera image (e.g. obtained by a 2D monochrome, color or similar conventional camera), a substantial increase of image intensity (e.g. when using a passive or active infrared camera), a substantial change in average color value, a substantial decrease of the average distance from the camera to points in the scene (e.g. of the average depth value per pixel over a depth image), and/or another similar simple detection strategy. Optionally, such change detection may be configured to detect a temporary change (e.g. a drop) that is associated with a return of the tested parameter (e.g. the average image intensity) to about its former value (e.g. to about the value as before the drop). An appropriate threshold, a fraction or another suitable test parameter for generating the trigger signal based on such 'substantial' change can be easily determined by the skilled person, e.g. by straightforward experimentation and/or trial-and-error.

It will be understood that for some gestures, a (short) delay timer may be activated (or a short delay may be otherwise accommodated) to allow the operator to vacate the camera's view before the trigger is generated in response of the detected gesture. A gesture may also be detected via a separate camera, e.g. different from the camera system. Gestures may also be detected in other ways, such as by an accelerometer or machine-detectable tag attached to the operator's clothing, e.g. to a wristband or in/on a clothing clip.

The method may comprise generating the trigger by acquiring an image, e.g. a live image stream, of the camera system and performing 110 a scene analysis. Thus, a suitable point in time may be automatically determined for which the patient is deemed to be in a suitable position for the diagnostic imaging procedure, i.e. when the preparation step of positioning the subject is considered to be finished. This does, however, not require detailed knowledge of the procedure to be performed. Particularly, the scene analysis may detect a point in time when the operator has left the immediate vicinity of the patient. It is an advantage that such smart auto-triggering may facilitate the patient movement detection, provided by embodiments of the present invention, without any need to change the diagnostic imaging workflow (e.g. except of course when substantial movement is afterwards detected by the method and the operator is implicitly or explicitly alerted to take action in response). The scene analysis may comprise detecting a shape of the operator and/or an extremity of the operator (e.g. one or both arms) in the (e.g. live stream) image acquired by the camera system, and generating the trigger when this shape has moved outside the image frame, e.g. after a short predetermined delay to avoid false detection. It will be understood that, for example, the arm or arms of the operator may generally move from a central position in the image frame toward and outside the edge of the frame. For example, the body part(s) of the subject and of the operator may separate spatially from each other at some point, and one (or multiple) spatially isolated segments may join the edge of the image frame (if not the case already) and decrease in size over time to zero (moving outside the frame).

This detection may be even as simple as detection a drastic decrease in the image content (e.g. pixels, surface areas or voxels) that can be attributed to living body matter. For the latter, it may be advantageous (but not strictly necessary) to use depth imaging (for a clear separation of the patient and operator from the background by differences in depth) and/or infrared imaging (using the heat emission of the body/bodies to obtain a good contrast for algorithmic separation from the background).

Additionally or alternatively, the camera system may comprise one or more additional cameras to cover a larger volume of the scene, e.g. a wider view of the examination room, e.g. set up to provide a long(er) image shot, such that the primary camera(s) can track the position of the subject in detail, while the secondary camera(s) can detect when the operator leaves the vicinity of the subject (e.g. thus as observed from the wider overall view). Worded differently, the trigger may be generated by recognition of a "gesture" of the operator (see above), in which the "gesture" may comprise or consist of leaving the volume of space monitored by (at least one camera of) the camera system.

Additionally, or alternatively, the trigger may be generated by proximity or spatial position detection of the operator using one or more sensors, e.g. an radiofrequency identification tag (on the body of the operator; RFID) in combination with a RFID reader positioned in or nearby the region of space where the patient is positioned for the examination. This may be used to detect when the operator is no longer present near the subject (or the state changes from present, i.e. RFID detected, to not present, i.e. RFID no longer detected), e.g. relying on the generally short range of RFID communication. Other suitable sensors may include, for example, a light beam gate (detecting when the operator crosses one or more light beams while leaving the examination region), an indoor positioning system (e.g. using position triangulation of the operator; e.g. which may be referred to, even though not entirely accurate, as a "GPS for indoor tracking"), sonar, lidar and/or radar technology, and/or another such suitable sensor system.

It is an advantage that interactions of the operator, e.g. actions to be performed by the diagnostic imaging technician, may be reduced and/or their workflow may be simplified. Ideally, interaction steps are reduced to a minimum. Any type of trigger to be given explicitly by the technician would increase the complexity of the workflow, even if it is only in a minimal way. A voice or gesture interaction may be preferrable to e.g. the push of a button, since it can be given without constraining the position of the operator to the location of the button. Likewise, automatic detection of the trigger without requiring a conscious action of the operator may be still more preferable, e.g. using detection of the position of the operator (particularly, of the moment when the operator leaves the region where the subject is set up for the examination).

The method may also comprise evaluating a trained machine learning and/or artificial intelligence model, e.g. an artificial neural network to generate the trigger (or: to take the output of the ML/AI model into account for generating the trigger). For example, an image or image sequence (e.g. a live stream) acquired via the camera system may be provided as input (directly, or indirectly, e.g. via intermediate processing such as a feature extraction step) to the trained ML/AI model. Thus, the step of performing 110 a scene analysis may comprise evaluating the trained machine learning and/or artificial intelligence model. However, other input(s) may also be used (additionally or alternatively; directly or indirectly), such as motion sensors, (e.g. X-ray) device control inputs and/or outputs, sound inputs (e.g. microphones), etc.

The ML/AI model may be (or have been) trained on a dataset that comprises a plurality, e.g. generally a large number, of user manipulation interactions with the diagnostic imaging modality, e.g. using an X-ray system (e.g. the same or similar type of system as to which the intended application of the method in accordance with embodiments relates). For example, the positioning of patients may be monitored, e.g. via the camera system, for a large numbers of test cases, and the time at which the patient has been positioned in the position that is to be maintained during the diagnostic imaging session can be added as an annotation (e.g. determined via a manually generated explicit trigger signal provided by the operator, or added post facto when preparing the training dataset). Thus, a ML/AI model can receive this training input (e.g. the camera images and/or other sensor data and/or information derived therefrom, e.g. extracted features) and the corresponding training output (trigger annotation) to be trained so as to generate the trigger at or sufficiently near an appropriate timepoint. It will be clear that the specific choice of input to the ML/AI model can be flexible, e.g. many input sources may be conceivable, in so far that sufficient information is at least implicitly deducible therefrom.

For example, user interactions with a diagnostic imaging (e.g. X-ray) system may be used as input (possibly in combination with other data, e.g. the camera data described above) to predict when the system (and user) are in a stable state, i.e. when the subject has been prepared for the diagnostic imaging session. For example, the ML/AI model may be trained to use implicit information, which may be embedded in actions for the repositioning of an X-ray tube, adjustment of beam parameters, movement of actuated support aids (e.g. a patient platform, ...), adjustment of collimation, the activation/deactivation of an indicative light field (visual representation of the X-ray beam), etc., to detect when the operator has finished the patient preparation procedure and moves on to other tasks, i.e. when the trigger should be generated.

Furthermore, it will be understood that the trigger that is effectively used in performing the method may also be determined by combining trigger signals from a plurality of sources and/or generation methods. For example, different inputs, such as a generic human interaction interface, a button, a voice control interface, a gesture detection system, a proximity/position detection system (e.g. to detect whether the operator and subject are near to each other), and/or other trigger signals may be combined, e.g. in a logical "OR" combination, in a logical "AND" combination, in a combination thereof (e.g. combining different logical operations in a non-trivial expression), by a weighted combination, by majority voting, and/or in any other suitable way.

The method further comprises, when said trigger is received 101, acquiring 102 reference image and/or spatial data of the subject using a camera system. The camera system is generally aligned to the anatomy of interest of the subject, e.g. such that the relevant body part(s) of the subject can be sufficiently observed by the data provided by the camera system. However, as discussed in detail hereinbelow, an accurate positioning and/or alignment of the camera is not necessary, e.g. it is not required to reproduce or determine an accurate mapping between the camera system's coordinate system and the diagnostic imaging system's coordinate system.

For example, the reference image/spatial data may be acquired at the moment of receiving the trigger or a predetermined time thereafter, e.g. preferably substantially simultaneously or shortly thereafter (for example, only delayed by a time that is needed for processing the trigger and controlling the camera system accordingly to acquire the data, and/or by other such insubstantial delays in view of technical and practical limitations). It will be understood that the camera system may also be configured to continuously capture image and/or spatial data (e.g. updating an image and/or spatial data buffer), such that, upon receiving the trigger, image and/or spatial data may be selected as said reference image and/or spatial data from the buffer, even though the selected data could be representative of a state of the subject (shortly) before the trigger is received. It will be understood that this approach allows a reference state of the subject to be captured (i.e. to be represented by the reference image/spatial data) that the subject is in at substantially the same time that the trigger (e.g. given by the operator) indicates the reference to be recorded, e.g. a buffer can compensate for any short technical delays, and may even compensate for any non-technical delay, e.g. a biological decision and/or muscular response time of the operator. However, it will also be understood that such technical and/or biological delays are typically of an order of magnitude that might not require any compensation at all, e.g. for most applications one or even a few seconds of delay (negative or positive, e.g. before or after the trigger time) may be generally acceptable.

Thus, "when said trigger is received" should be interpreted in the sense that the acquired reference image/spatial data is representative of the state of the subject (e.g. having a certain spatial position, orientation and/or other geometrical state in time) at substantially the time that the trigger is received (and thus, also, at substantially the time that the trigger is generated), e.g. less than 5 seconds before or after the time of receiving the trigger, e.g. preferably less than 1 second before or after said trigger time, preferably in the range of half a second before to half a second after receiving the trigger.

After positioning the patient, in which the operator has checked (or actively guided the patient) that the spatial configuration of the patient (e.g. relatively to the diagnostic imaging system) is correct, e.g. is (sufficiently) suitable for the examination to be performed, the trigger is generated (explicitly by the operator, implicitly by movement of the operator, or automatically by a dedicated algorithm), and the reference image/spatial data is determined in response, e.g. a camera image(s) of the subject, or, at least of the anatomical area of interest, is taken, i.e. at a time sufficiently close to the moment in time that was indicated by the trigger, which is assumed to have the subject positioned in the correct (i.e. the desired) pose.

Acquiring 102 the reference image and/or spatial data of the subject using a camera system may comprise a conventional (digital) photo acquisition, e.g. capturing a color or monochrome camera image. It may also be advantageous to use infrared imaging, e.g. such that the camera system comprises an infrared camera, e.g. a camera configured to obtain infrared image data (possibly in combination with one or more visible spectrum components). The camera system may thus comprise one or more optical cameras, e.g. sensitive to one or more spectral bands in the visible (and/or infrared) range, e.g. monochrome camera(s) and/or color camera(s). The color camera(s) will be understood to refer to (a) color camera(s), without necessarily limiting to a specific choice of color components (e.g. RGB) or choice of their specific combination. It will also be understood that the "color" camera may also be (unintentionally or intentionally) sensitive to one or more infrared bands, possibly exclusively so, but also possibly in combination with visible light bands. The camera system is also not necessarily limited to "color" cameras with a relatively small number of color bands (e.g. Red, Green, Blue), but may also comprise (a) multispectral camera(s), e.g. which is adapted to quantify the light spectrum received at each pixel location more fully, e.g. by decomposition into a relatively large number of spectral bins (e.g. such that a substantial spectral image dimension is formed in addition to the 2D image coordinates).

The reference image and/or spatial data, e.g. one or more images and/or spatial information about the observed scene (containing the subject, or relevant body part or parts thereof) in any suitable form, may optionally comprise 3D and/or depth information. Thus, acquiring 102 the reference image and/or spatial data may comprise acquiring the 3D and/or depth information. For example, acquiring the reference image/spatial data may (alternatively or additionally) comprise the acquisition of a depth image and/or the acquisition of three-dimensional (3D) data (e.g. a 3D point cloud and/or a surface model constructed from such 3D point cloud) in another manner by the camera system. For example, the camera system may comprise a depth camera, and/or another suitable device for capturing 3D surface information, e.g. capturing a 3D (exterior) surface map of the patient's body (or, at least, of the relevant body part/parts). Thus, the image and/or spatial data may comprise a monochrome image, a color image, an infrared image, a multispectral image, a depth image, a 3D image and/or a combination thereof. Depth information may be gathered in addition to in-plane image information, or, at least, three-dimensional (3D) information may be acquired that is not trivially reducible to 2D data, e.g. not solely expressed in coplanar coordinates. For example, a 2D map (e.g. image) of depth, and/or a set of 3D points (e.g. a 3D point cloud) may be acquired. A depth image may for example be obtained by a range camera, which may produce a 2D image that shows the distance to points in a scene from a predetermined reference point or plane (e.g. a focal point or other reference point). Depth information may be obtained by stereo imaging, in which images are (e.g. substantially simultaneously) acquired by two (conventional, e.g. monochrome or RGB) cameras from different vantage points. Processing techniques known in the art may be applied to such stereo images (to the pair of concomitantly acquired images) to derive depth information therefrom, e.g. by parallax analysis. This principle can readily be extended to more than two cameras, which commonly may also be referred to as stereo imaging (i.e. "stereo" is not to be interpreted in the narrowest sense of using only two cameras). An advantage of stereo (or multi-camera) depth imaging is that the needed device components may be easily obtainable and may be relatively cheap. For example, such system may be constructed using conventional (2D) cameras, or an existing (2D) camera may be easily upgraded by adding one or more further cameras.

The processing that may be required for calculating the corresponding depth pixel maps (i.e. depth images) is relatively simple, and can be easily implemented in software and/or hardware (potentially leveraging graphical processing unit, GPU, processing, and/or dedicated hardware, e.g. application-specific integrated circuits and/or field-programmable gate arrays). An advantage of stereo or multi-camera depth imaging is that the scene, e.g. comprising the subject, can be imaged without purpose-specific lighting and/or other direct manipulation. In other words, the scene can be imaged, and processed to determine the depth data (i.e. the third dimension in addition to the planar image coordinates), without actively interfering with or influencing the scene, i.e. essentially passively, except for, possibly, generic (general-purpose) lighting.

Depth images may also be acquired by active techniques. For example, a sheet of light may be scanned over the scene to image its reflection. From the shape and displacement of the imaged (line) reflection, the distance between the reflections (points on said line - e.g. every point in the image after fully scanning the scene) and a reference, e.g. the light source and/or camera can be relatively easily computed, e.g. using triangulation methods. Another type of active depth imaging that may be applied uses a structured light 3D scanner, which may be seen as a more intricate version of the light sheet triangulation mentioned hereinabove, with additional advantages such as requiring fewer (or none) physical displacement(s) of the camera and/or light source to fully qualify the depth values of the scene.

Depth information may be acquired by a time-of-flight camera, LIDAR or RADAR system. A possible advantage of time-of-flight camera depth imaging is that an image may be collected substantially instantaneously, e.g. without scanning a point, line, structured light pattern, laser, wave or generally any "active" (e.g. time-varying, e.g. scanning) analyzer over the scene. Other potential principles of depth imaging may include interferometric techniques (e.g. relying on coherent light), coded aperture imaging and/or possibly other techniques.

Furthermore, acquiring 102 the reference image/spatial data may also comprise the (e.g. substantially simultaneous) acquisition of images (e.g. conventional 2D images and/or depth images and/or 3D data) of the subject from different viewpoints (e.g. camera vantage points). The camera system may thus also provide any of the aforementioned data, simultaneously, from different vantage points, e.g. such as to acquire image/spatial data from different camera positions (e.g. using different cameras in the camera system), for example to view the subject from at least two different angles. Thus, even if no depth or 3D data is used, explicitly, monitoring of the subject's position may still be possible by detecting movement in (any of) at least two different image planes.

It will be understood that the comments with respect to the image/spatial data hereinabove equally apply to the reference data as to the live tracking data discussed further hereinbelow. The reference data and live tracking data are generally of the same type, nature and configuration, in view of being acquired by the same camera system and preferably with the same configuration settings thereof.

It is noted that it is not necessary that the camera system is connected to the diagnostic imaging system (e.g. an X-ray system), e.g. no mechanical, electronic and/or data processing integration of the camera system into the diagnostic imaging system is required. The position and orientation of the camera system (or of individual camera components thereof) need not be determined precisely with respect to the diagnostic imaging system (e.g. with respect to a radiation beam axis of a projection X-ray system), it merely suffices that the subject, or the relevant anatomy of the subject, is observable by the camera, e.g. falls within the image frame. Therefore, no intricate calibration and/or alignment procedures are required. Also, the camera system does not require any input from the diagnostic imaging system, nor needs to provide an output to the diagnostic imaging system, e.g. an input/output via electronic signaling and/or interaction between software of the respective systems. Since the method does not necessarily explicitly determine the position of the subject with respect to the diagnostic imaging system's coordinate system, e.g. to check whether the position is correct for a specific examination, an accurate positioning and/or alignment of the camera system is not strictly necessary, nor a calibration of the camera coordinate system with respect to the coordinate system of the diagnostic imaging system.

The method 100 further comprises, after said reference image and/or spatial data of the subject is acquired 102 using the camera system, (e.g. repeatedly 114) acquiring 103 a further image and/or spatial data (e.g. which may also be referred to as live tracking data) of the subject using the camera system, e.g. periodically or substantially continuously acquiring the further image/spatial data, for example by video monitoring (e.g. such that the further image/spatial data may correspond to, or may comprise, a most recent camera image in a live video stream or streams).

The method also comprises (e.g. also repeatedly) comparing 104 the reference image and/or spatial data of the subject, which represents the subject's state at substantially the time that the trigger was received, to the further image and/or spatial data of the subject, which represents a more recent state of the subject. For example, the further image and/or spatial data may be substantially continuously compared to the reference, e.g. compared at a reasonable frequency, which may also correspond to the sampling frequency at which the further image/spatial data is updated (e.g. at least 0.1 Hz, preferably at least 1 Hz, e.g. at least 10 Hz). Even if the further data is continuously updated, or at least with some frequency to enable a more or less fluent monitoring of the subject's present state via the camera observations, it will be understood that this does not necessarily need to imply a constant refresh frequency, even though, in a typical approach, conventional video capture and processing frequency may be advantageously used to update the further image/spatial data at such a constant refresh rate (frames per second, FPS), e.g. in the range of 0.1 Hz to 120 Hz, e.g. 10 Hz to 80 Hz, e.g. 20 Hz to 60 Hz, e.g. 25 Hz, 30 Hz; 40 Hz or 50 Hz, without any limitation to these illustrative ranges and/or values.

Thus, any (relevant and/or substantial) movement of the subject can be detected in a simple and effective manner by comparing the current image and/or spatial data to the reference data.

The method comprises providing 105 an output to the operator and/or to the subject that is representative of this comparison of the further image and/or spatial data (e.g. the current image, e.g. the most recently acquired image) to the reference image and/or spatial data to indicate movement of the subject (e.g. of at least one body part of interest of the subject) with respect to the earlier state of the subject represented by the reference image and/or spatial data. Thus, attention of the operator and/or subject may be drawn to undesirable movement of the subject (e.g. a patient) before commencing the diagnostic (e.g. medical) imaging procedure after initial preparations to bring the subject into the correct pose (represented by the reference data) were completed. The output may be presented to the operator (and optionally also to the subject) via (a) display monitor(s) or other suitable human interface device(s). Preferably, the output may be dynamic, e.g. by repeatedly updating the output based on a live data stream form the camera system. For example, the information to be outputted may be shown on the display in a graphical representation (e.g. an overlay and/or other type of visualization), by (a) simple numeric value(s), by a text string and/or in another suitable form.

Providing the output may also comprise displaying a result of said comparison, e.g. in a continuously (e.g. with some reasonable frequency) updated display format. For example, a difference image may be displayed to the operator (and/or directly to the subject to allow the subject to autocorrect his/her position). Many different ways of visualizing this difference and/or the result of said comparison may be envisioned. For example, the further image and/or spatial data may be displayed on a display monitor (e.g. to the operator and/or to the subject) on which the difference image (e.g. obtained by image subtraction) may be shown in an overlay, e.g. as a color overlay. Thus, differences of the currently observed state with respect to the reference state of the subject can be highlighted, e.g. by color to mark the differences. It will be understood that this may also be achieved by different types of overlay, such as changing the image intensity instead of color (e.g. literally highlighting differences), or creating a contour overlay to indicate a contour of the difference region. For example, a perimeter of an image region where the image difference exceeds a predetermined threshold may be marked by a line, e.g. a solid or dashed line.

An approach is provided (e.g. enabled) by embodiments of the present invention to aid in positioning the patient for a diagnostic imaging examination, and that allows the detection of movement of the patient regardless of the intended examination, e.g. by not requiring an explicit definition of the 'correct' pose/positioning (e.g. other than the recorded reference image of the subject after being guided into this non-explicit state by the operator), and/or regardless of the patient's body (e.g. a specific body shape, anatomical parameters, possibly specific medical conditions, ...). In principle, embodiments of the present invention don't even need to assume that the patient is human, e.g. a method or device in accordance with embodiments can be suitable for assisting in the positioning of a human patient (e.g. in a typical medical diagnostic application), but can also, without any modification, be used for assisting in the positioning of an animal patient (e.g. in veterinary medicine). Even though a system that can easily switch between human and veterinary medicine might not find broad application, or might even be of very limited use in practice, this clearly illustrates the versatility of the approach. For example, the method in accordance with embodiments can be used for a patient that has a non-standard body shape or features, e.g. as the result of amputation, a congenital condition or the like, without requiring reconfiguration and/or modification.

However, in a method in accordance with embodiments of the present invention, comparing 104 the reference image and/or spatial data of the subject may also comprise detecting 106 at least one image feature and/or landmark in the reference image and/or spatial data as well as (the corresponding feature or features) in the further image and/or spatial data. Thus, a relative displacement of the at least one image feature and/or landmark between the reference and further image and/or spatial data may be determined for use in determining the output. For example, at least one anatomical feature and/or landmark may be automatically detected (e.g. detected and located as function of the camera image coordinates) in the reference image and/or spatial data as well as in the further image and/or spatial data. The anatomical feature and/or landmark may, for example, comprise one or more predetermined landmarks on one or more body parts, such as a landmark(s) on a shoulder, a hip, a wrist, a finger (e.g. in the general sense, or a specific finger, e.g. the little finger or the thumb), a knee, an ankle, a toe (e.g. generally, or a specific toe) and/or other anatomical parts of the body. The landmark(s) may comprise a point (or, by extension, a line segment and/or surface patch) that is (e.g. easily) recognizable by automatic processing, e.g. with a high specificity and/or sensitivity. Such point may be a salient feature of a specific body part, e.g. the easily identifiable ulnar head protrusion of the wrist, a center point of a knuckle and/or other such visible anatomical features. Such salient features can for example be easily detected in images by a suitable algorithm, e.g. by a combination of image processing filters tuned to the specific shape, orientation, boundary shape and/or other visual (and/or, generally, spatial) characteristics of the body part (and/or a specific region or part thereof, e.g. the immediate vicinity of the salient feature to detect), by a trained machine learning model, and/or any other suitable method known in the art.

For example, the method (and/or device) in accordance with embodiments may be adapted to detect one or more specific image features (corresponding to the anatomical feature and/or landmark), and to detect and/or quantify movement of the subject based on displacement (and/or change of another spatial variable or variables, e.g. orientation) of such image feature, e.g. a change of position of the anatomical feature and/or landmark in the further image and/or spatial data relative to the position of the same feature and/or landmark in the reference image and/or spatial data.

While the automatic detection of such at least one anatomical feature and/or landmark might be seen to reduce the versatility, e.g. the substantially universal applicability for patient positioning assistance in diagnostic imaging as discussed hereinabove, it is noted that this is not necessarily the case, or may only be so in a limited sense. For example, an embodiment may also be configured to fall back to a simpler approach to quantify (and/or detect) movement, e.g. by image subtraction (further image vs. reference image) and/or calculating at least one measure based on the subtraction image, e.g. a maximum absolute difference, a mean absolute difference, a predetermined percentile of the (e.g. absolute) difference, a sum of squares, and/or any other such value to characterize a magnitude of the overall displacement. However, when (a) landmark feature(s) can be detected, the detection of movement may take changes in position of such landmark into account to be more sensitive to relevant motion as opposed to any arbitrary change in image content, and/or the position of the landmark(s) may be indicated on an (e.g. overlay) image based on a simpler image comparison (e.g. based on a subtraction image) to further enhance the ease by which the operator can ascertain that the correct position of the subject is maintained. It is also to be noted that such landmark features may be used to easily add 3D information to a 2D representation, e.g. a 2D (image) representation may easily show displacement of the subject in the image plane (e.g. by accenting change with respect to the reference using a color overlay), while the landmark position may also be indicated on the visual representation together with an indicator that shows the depth (out-of-plane position) of the image feature and/or its relative change with respect to the reference.

The method in accordance with embodiments may be adapted to detect at least one anatomical feature and/or landmark, e.g. a set of different features and/or landmarks, without necessarily relying on (i.e. requiring) the presence of each feature and/or landmark, or on the presence of a specific subset or combination of the anatomical features and/or landmarks, in any specific reference image/spatial data. For example, one or more detection algorithm may be used to detect various different anatomical landmarks, e.g. at least one salient point for each joint, bone and/or other anatomical part of interest, such that, regardless of the context of a specific examination, at least one, or at least a few, of the supported landmarks is likely to be detectable (and hence detected). Thus, regardless of whether, for example, the patient's arm or the patient's head is being examined, at least one or at least a few points may be detected that (with a predetermined likelihood) correspond to points that are relevant for the positioning of the underlying anatomy. For example, for an imaging examination of the arm, bony protrusions on the wrist and elbow may be detected, in which a detected displacement of at least one thereof is likely associated with (substantial) movement of the arm as a whole. Likewise, for the head, landmark points on the chin, on the top of the skull, the frontal tip of the nose, and/or other such easily detectable points may be used. The applied algorithm does not necessarily need input to define the correct set of landmarks to search for, e.g. the algorithm may attempt to detect all landmarks and only retain those for which the detection does not fail, or for which an estimated detection accuracy is sufficiently high. The landmark detection may also be performed by a trained machine learning algorithm, such that the model's training can implicitly take co-occurrence of specific features into account to determine a set of detected features that is internally consistent (e.g. corresponds to a combination that was typically encountered in its training data).

The landmark/feature detection process is preferably agnostic, e.g. preferably does not take a specific geometric model of the subject and/or body part(s) of the subject into account. It is to be noted that such a landmark detection algorithm may be adapted to detect landmarks (e.g. in any combination from a library of supported landmark features), without requiring a manual selection of the procedure or any prior knowledge of the specific procedure being performed. In fact, even if incorrect points are identified, e.g. a landmark is detected that was intended as a center of the patella in a reference camera image of the elbow of the patient, this does not need to imply a problem. In so far the detection of this feature and/or landmark point is sufficiently stable, i.e. such that the same point can be detected in the further image/spatial data, patient movement can still be sufficiently accurately qualified and detected by changes in the position of this landmark point, even if less anatomically relevant.

In fact, in accordance with embodiments of the present invention, an approach for detecting 106 images features and/or landmarks may be used that relies on a good detectability and saliency of the image feature, and not necessarily on a predetermined definition of a corresponding anatomical feature. For example, a filter or combination of filters may be applied to detect the center of more or less homogeneous areas, e.g. of a certain size or dimensions (e.g. in a certain size range). While such approach may be tuned to detect the center of the patella specifically, it may also be included as a generic feature detector for the sake of its simplicity and robustness.

If the optional detection of at least one anatomical feature and/or landmark is applied in accordance with embodiments, the position of the, or each of the, at least one anatomical feature and/or landmark may be compared between the further image/spatial data and the reference image/spatial data to detect movement of the subject. In other words, the step of comparing the reference image and/or spatial data of the subject to the further image and/or spatial data of the subject, may comprise a comparison of the (or each) anatomical feature and/or landmark, e.g. a comparison of the position thereof, in the further image/spatial data versus in the reference image/spatial data. If a plurality of features are detected, motion may be characterized by a sum, a maximum, a minimum, and/or (an)other summary statistic(s) of the individual displacements (e.g. a displacement for a specific feature being expressed by the length of the displacement vector). A more direct image comparison, e.g. using a difference image, may also be used alternatively (e.g. not requiring any landmark detection), or in combination with such landmark-based approach.

For example, a maximum absolute image difference or a maximum displacement vector length for an optical flow map calculated to compare the reference vs. the further image may be used as fallback when no landmarks were detectable may be used as fallback when no landmarks were detectable. A combination of landmark-based and direct image-based motion quantification may be used, e.g. expressing a value indicative of (e.g. a magnitude of) motion of the subject by a weighted sum or other type of combination of these individual measures.

Thus, a substantial change in the position of a point(s) indicative of the position(s) of, e.g., anatomical structures or landmarks such as shoulders, hips, wrists, fingers, knees, ankles, toes etc., may be automatically detected. The output may show the (corresponding - i.e. relating to the same landmark) currently detected position (in the further image/spatial data) and the reference position (in the reference image/spatial data) for the or each detected landmark, and/or the difference between said position(s), e.g. a magnitude of displacement of a landmark.

Providing 105 the output may also comprise determining a difference measure indicative of movement of the subject relative to the reference state based on said comparison 104, and, when said difference measure exceeds a predetermined threshold, alerting the operator that the subject is no longer in the intended reference position, e.g. has moved substantially relative to the established reference data. Thus, the difference measure may be based on direct image subtraction (or other type of image comparison calculation) and/or on the quantification of a change in position of the detected image feature(s) and/or landmark(s).

As discussed hereinabove, the location and/or amount of movement can be displayed by using an image overlay. Fig. 2 shows an illustrative visualization of patient movement with respect to a frozen reference image of the (correctly) positioned patient. It is to be noted that, due to the limitations of a grayscale representation, the effect of a color overlay is not represented accurately in Fig. 2. The difference region is therefore, alternatively, shown by a shadow region 21 with contour line. While such representation may be used in practice, in accordance with embodiments of the present invention, it will be understood that a color overlay (with a different color, e.g. a transparent color of sufficient saturation on a monochrome source background image, e.g. the current camera image in a live stream) may be a more convenient form of representing such overlay in practice.

Furthermore, anatomical structures or landmarks, or even just salient image features (e.g. points and/or areas in the image which can be detected in a stable manner throughout an image sequence, e.g. in a video stream, such as local extrema, cusp points, centers or centroids of relatively homogeneous areas, etc.), may be automatically detected (cf. hereinabove). The location of such landmarks may be shown, separately and/or in the overlay, e.g. both the current position (determined in a live stream) and the reference position, a vector indicating the displacement between the reference and current position, and/or in another suitable form. Alternatively and/or additionally, a substantial change of the image (current vs. reference) and/or of the landmark position(s) may be used to detect a substantial patient movement automatically, e.g. to alert the operator that such a substantial movement has occurred.

When depth and/or 3D data is used (e.g. acquired by a depth, stereo and/or 3D camera system), a patient movement can also be detected when the depth (e.g. the coordinate of a landmark point in the normal direction to the image plane) changes, e.g. even when the movement in the image plane is below a detection threshold (explicitly or implicitly by evaluation by the operator). Furthermore, the detection of salient points, e.g. representative of anatomical features, may be more robust and easier to calculate when depth and/or 3D data is used. For example, convex and/or concave regions (of the 3D body surface) may be detected and the center thereof may be used as a (likely relevant) landmark. Thus, the tip of an elbow, the tip of the nose, the center of the chin, the visible ulnar head at the wrist, and/or other such anatomical features may be easily detected by a simple approach. Likewise, saddle points may be anatomically relevant and/or may allow a robust detection of the same point on the body's surface under movement (or, at least, under minor movement, assuming that the subject intends to remain still, as typically required for the diagnostic imaging procedure).

A similar effect may be achieved by 2D imaging in two image planes that are not coplanar, e.g. presenting and/or analyzing and/or visualizing two orthogonal (without limitation thereto) views of the subject acquired from at least two different cameras.

The output allows the operator to make an objective decision, before the actual diagnostic image acquisition, whether a repositioning of the subject would be necessary for obtaining a good diagnostic (e.g. X-ray) image. This decision can be made by the operator, or automatically, e.g. by a simple decision algorithm (e.g. a metric expressing the magnitude of motion, e.g. the difference measure discussed hereinabove, exceeding a predetermined threshold) and/or an artificial intelligence based algorithm, e.g. a trained machine learning model.

In summary, the method may acquire at least one reference photographic image and/or depth (or 3D) image (or, at least, spatial data, e.g. 3D surface data in the form of a 3D point cloud or surface mesh), when the operator or a controller decides that the positioning setup is finished (the reference data being acquired at substantially a time that the trigger, discussed hereinabove, is received). Then, the actual video stream from the same camera system can be (e.g. continuously) compared with the reference data, and, e.g., shown in an overlay. The movement may also be quantified and provided as an output, e.g. generating an alert when substantial movement away from the reference has occurred. The deviation of the current camera image(s) and the reference image(s), indicative of a "good" positioning of the subject, may be evaluated by an AI system, a conventional algorithm and/or may just be displayed on a monitor for an objective decision by the operator when the diagnostic image acquisition is to be executed on whether a repositioning of the patient would be necessary.

The output may, for example, be provided via a display monitor of a console computer (e.g. outside the acquisition room) for controlling the diagnostic imaging system, or via a separate monitor dedicated to this purpose (e.g. thus avoiding the need for any integration with the diagnostic imaging system whatsoever).

The operator (e.g. a medical technical assistant, MTA) can be signaled explicitly and/or implicitly (by allowing the operator to compare the states in a convenient data presentation, e.g. an image overlay) when the deviation from the original spatial configuration (frozen in the reference data) is too large to proceed with the diagnostic image acquisition.

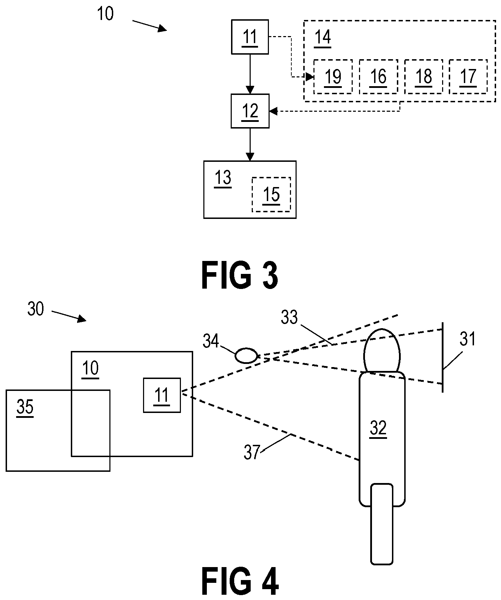

In a second aspect, the present invention relates to a device for detecting movement of at least one body part of a subject in a diagnostic imaging examination. Fig. 3 schematically illustrates a device 10 in accordance with embodiments of the present invention. For example, the diagnostic imaging examination may comprise an X-ray imaging examination, e.g. a projection radiography examination. The device 10 may thus be comprised in a diagnostic imaging system, e.g. an X-ray imaging system, or in a workstation for such system. For example, the X-ray imaging system may be an X-ray projection radiography system.

The device 10 may be adapted to detect a change in position, orientation and/or other spatial configuration of the subject, or at least of the subject's anatomy that is relevant for the examination, with respect to a reference state of the subject, e.g. as positioned correctly for the examination. Thus, the device may be adapted to assist in patient positioning in a preparation step of the imaging session, e.g. by checking for undesirable movement of a subject (e.g. a patient) after initial preparations to bring the subject in a correct spatial configuration for the examination are completed and before actually executing the diagnostic imaging acquisition. For example, an operator (e.g. MTA) may bring the patient into a position (generally, a spatial configuration) that is deemed suitable or desirable, e.g. optimal, for the intended diagnostic imaging examination.

The device 10 may comprise a processor, data storage memory, input(s), output(s), a user interface and/or other means generally known for performing a method as discussed hereinabove, e.g. when programmed and/or configured accordingly. Thus, the device may comprise a computer and a computer-program product in accordance with embodiments of the present invention (i.e. adapted to be executed by the computer). Additionally or alternatively, the device may comprise hardware specifically designed and/or configured to perform a method in accordance with embodiments of the first aspect of the present invention, e.g. comprising an application specific integrated circuit and/or configured field-programmable gate array to perform a method in accordance with the first aspect of the present invention. The device may comprise a computer. The device may also comprise a plurality of processors, computers and/or other processing devices acting collaboratively, e.g. using a host-client architecture, web-based architecture, cluster processing, and/or other form of distributed processing, data collection, data presentation and/or data storage.

The device comprises a processor 12 and an output 13. The device also comprises an input 14 for receiving a trigger to indicate that a current spatial configuration of the subject is to be maintained for the diagnostic imaging examination.

The input 14 may comprise (or may be comprised in) a human interaction interface, e.g. may comprise a button 17, a voice control interface 18 and/or a gesture detection system 19 to detect a gesture made by the operator. For example, once the subject is in the desired position, the operator may provide the trigger to indicate that the current position (i.e. spatial configuration) of the subject is to be maintained. It will be understood that a gesture detection system 19 may comprise the camera system 11, discussed hereinbelow, in combination with suitable processing (gesture detection) performed by the processor 12. Likewise, the voice control interface 18 may comprise a microphone in combination with suitable processing (voice command recognition) performed by the processor 12.

The input 14 may comprise a proximity and/or position detection system 16 to detect proximity of the operator to the subject. For example, the proximity and/or position detection system 16 may comprise a RFID tag, on the body of the operator, in combination with at least one RFID detection sensor (near the subject, i.e. in, at or near the examination region of the diagnostic imaging system). The proximity/position detection system 16 may comprise at least one light gate, to detect when the operator leaves the vicinity of the subject (i.e. the examination region). The proximity/position detection system 16 may comprise a sonar, radar and/or lidar system, and/or another sensor system for presence, proximity and/or position detection of the operator. Thus, the trigger may be generated when the proximity detection indicates that the operator has left the vicinity of the subject, or the position of the operator is detected to be substantially away from the subject, e.g. at a distance that renders direct physical interaction of the operator with the subject impossible and/or impractical for the purpose of assisting in positioning the subject. The proximity/position detection system 16 may also use the camera system (in combination with suitable processing by the processor) to detect when the operator leaves the examination area where the subject is set up for the procedure, e.g. when the operator leaves the camera image frame of the camera(s) for monitoring the position of the subject and/or is detected to be sufficiently remote from the subject in a camera image frame captured by a further (e.g. a long shot) camera that is dedicated to this purpose specifically. Thus, the processor may be adapted to perform a scene analysis of the live imaging stream from the camera system to detect a predetermined condition for generating the trigger and generating thje trigger in response, in which this predetermined condition corresponds to the operator leaving the immediate vicinity of the subject.

Additionally or alternatively, the input 14 may be adapted to receive the trigger from an automated system for detecting a predetermined reference spatial configuration of the subject, as required for the diagnostic imaging examination, in a live imaging stream provided by the camera system 11. Thus, a device for determining a suitable spatial configuration of the subject for a specific examination and for guiding the subject into the desired spatial configuration, as known in the art, may be used to generate the trigger. Since such automated system may be implemented by the same processing hardware, the trigger may be a virtual signal, e.g. communicated via a memory flag, file, web socket, or similar mechanism for inter-process communication. Such automated system may also be comprised in the device 10 in accordance with embodiments, but not necessarily so.

The device 10 may also comprise an artificial intelligence module (e.g. a machine learning processor core) for evaluating a trained machine learning model. For example, this AI module may be comprised in the automated system referred to hereinabove. The AI module is adapted to generate the trigger signal by taking an output of the evaluated model into account. The trained machine learning model uses, as input, the image and/or spatial data, and/or data derived therefrom, e.g. obtained from the camera and/or 3D surface scanning system 11, and/or data from the diagnostic imaging system, e.g. received via an interface with the diagnostic imaging system. Such data received from the diagnostic imaging system may for example comprise device state information of the diagnostic imaging system and/or control input information received by the diagnostic imaging system from user interactions. Thus, the AI module can detect (implicit) regularities in the use of the diagnostic imaging system that are indicative of the patient positioning step being completed. Such implicit regularities and relationships can be pre-learned by the model, e.g. based on a suitable set of training data. It will be clear that the AI module is not necessarily physically separate from the processor discussed hereinbelow, e.g. the processor 12 may be adapted to implement the AI module. Alternatively, the AI module may be a dedicated hardware module (e.g. an ML core) for evaluating the trained machine learning model, e.g. a trained neural network.

It will also be understood that the input may combine different sources of information to determine the trigger signal to relay to the processor. For example, different user interfaces and/or detection modules may be combined, the (trigger) output of which may be combined in any suitable (and/or user-configurable) manner to arrive at the trigger for use in the processing discussed hereinbelow.

The device 10 comprises a camera and/or 3D scanning system 11 for acquiring image and/or spatial data of the subject. The camera and/or 3D scanning system may comprise at least one camera arranged such as to obtain a two-dimensional and/or three-dimensional view of the subject, or at least of the relevant body part(s) of the subject. The camera and/or 3D scanning system may comprise at least one optical camera for acquiring a two-dimensional image. The camera and/or 3D scanning system may comprise a plurality of cameras, e.g. set up at different positions and/or so as to view the subject from different angles.

The camera and/or 3D scanning system may be adapted for acquiring depth information. The camera and/or 3D scanning system may comprise a depth camera. The camera and/or 3D scanning system may comprise a plurality of two-dimensional optical cameras to acquire (e.g. substantially simultaneously) two-dimensional images from a plurality of different vantage points. Thus, the camera and/or 3D scanning system (and/or processor) may be adapted to determine the depth information from the two-dimensional images, e.g. by applying an algorithm for depth inference from stereo or multi camera images.

The processor 12 is adapted for acquiring reference image and/or spatial data of the subject using the camera system when said trigger is received. The reference image and/or spatial data may be acquired at the moment of receiving the trigger or a predetermined short time thereafter, and/or the reference image and/or spatial data may be selected from a buffer storing a stream of image and/or spatial data acquired by the camera system, e.g. in which the selection corresponds to a (e.g. most) recent point in time before the trigger was received.

The processor is furthermore adapted to acquiring a further image and/or spatial data of the subject using the camera system after said reference image and/or spatial data of the subject is acquired.

The reference image and/or spatial data (as well as the further image and/or spatial data, which are generally of the same type and nature and typically correspond to the same settings configuration) may comprise a conventional monochrome and/or color digital photographic image, in the visible and/or infrared spectrum. The reference (and further) image and/or spatial data may comprise a plurality of simultaneously (or at least concomitantly) acquired images from different cameras, e.g. from different vantage points. The reference (and further) image and/or spatial data may comprise 3D and/or depth information.

The processor is adapted to compare the reference image and/or spatial data of the subject, which represents a reference state of the subject at substantially the time that the trigger was received, to the further image and/or spatial data of the subject, which represents a more recent state of the subject.

The processor may be adapted for comparing the reference and further image and/or spatial data of the subject by detecting at least one image feature and/or landmark in the reference image and/or spatial data as well as in the further image and/or spatial data, and comparing a position of the at least one image feature and/or landmark between the reference and further image and/or spatial data for use in determining said output. The image feature and/or landmark may comprise at least one anatomical landmark on a joint, bone, muscle and/or other externally discernable anatomical feature of the subject's body. The image feature and/or landmark may comprise one or more salient image features, e.g. such that the position of a point on the imaged object (the subject) corresponding to this salient image feature can be stably, accurately and robustly tracked in different images (particularly, in at least the reference image/spatial data and the further image/spatial data).

The processor may be adapted for comparing the reference and further image and/or spatial data of the subject taking three-dimensional information into account, e.g. by determining 3D points (or surface segments or space regions) for which the corresponding acquired depth (and/or 3D) information in the reference image/spatial data differs substantially from the corresponding depth (and/or 3D) information in the further image/spatial data. Such identified points (or surface or space regions) may be clustered or otherwise combined (e.g. summarized in a suitable data representation) to allow a 3D visual representation of the difference (e.g. in an output image and/or overlay) and/or to quantify the movement (displacement) of the patient in his/her current state relative to the reference state (e.g. for use as or in the difference measure discussed further hereinbelow).

The processor is adapted to provide output data to the operator and/or to the subject via said output 13. The output data is representative of said comparison of the further image and/or spatial data to the reference image and/or spatial data so as to indicate movement of the subject with respect to the reference state of the subject. For example, the shape, area and/or volume of the detected (2D or 3D) difference may be presented, i.e. visualized, as a (e.g. color) overlay and/or contour on the further image (e.g. on a most recently acquired image of the subject) and/or on a 3D model visualization of the scene (e.g. a 3D visualization of the most recently acquired image/spatial data of the subject). For example, the 3D exterior surface of the subject (or the relevant body part thereof) may be rendered and the difference region may be marked in a different color or with other properties (different from the property values used for the primary surface rendering) to highlight the difference. Where reference is made to an overlay display of the difference over the further (e.g. most recently acquired) image/spatial data, it will be understood that approximately the same effect can be achieved with an overlay of the difference over the reference image/spatial data, even though it might be preferable to visualize the live data stream with difference annotations (the overlay) for the sake of avoiding any loss of available information in the presentation to the operator due to processing artefacts.

The output enables the operator to make an objective decision, before the actual diagnostic image acquisition, whether a repositioning of the subject would be necessary to obtain a good diagnostic (e.g. X-ray) image. This decision can be made by the operator, or automatically, e.g. by a simple decision algorithm (e.g. a metric expressing the magnitude of motion exceeding a predetermined threshold) and/or an artificial intelligence based algorithm, e.g. a trained machine learning model, and/or a combination of the aforementioned.

The processor 12 may be adapted to repeat the steps of acquiring the further image and/or spatial data, via the camera system 11 (e.g. periodically and/or substantially continuously so as to obtain a live stream of further image and/or spatial data), comparing the reference image and/or spatial data to the further image and/or spatial data (e.g. comparing the most recently acquired further data to the reference data), and providing the output data. Thus, in each iteration, the output data may be updated to represent the current movement of the subject with respect to the reference state, e.g. such that a dynamic view is presented of the current change of spatial configuration of the subject with respect to the earlier reference state of the subject.

The device may comprise a user interface, e.g. which may comprise a display monitor, a mouse, a keyboard and/or one or more similar human interface devices known in the art. The user interface may be adapted to control the processes discussed hereinabove and below, e.g. to provide the trigger. The user interface may be adapted for interacting with the operator, e.g. a healthcare professional, a medical doctor, a nurse, an imaging technologist or the like. For example, the user interface may be used by the operator to control the device and/or monitor the positioning of the patient in the preparation phase of the imaging session.

The output 13 may, for example, comprise a display monitor 15. The processor 12 may be adapted for presenting, via the display monitor, a visual representation of the comparison. The processor 12 may be adapted for repeatedly performing the monitoring loop discussed hereinabove, so as to dynamically update the operator with respect to the position (i.e. spatial configuration) of the subject relative to the reference state.

The user interface may comprise the input 14, but the input 14 is not necessarily (only) physically at the same location as an output, e.g. a display monitor 15, of the user interface.

The processor may be adapted to provide as said output data, or part of said output data, an image overlay that shows said comparison in the form of a difference image as an overlay, e.g. a color overlay, over said further image and/or spatial data.

The processor may be adapted to determine a difference measure indicative of movement of the subject relative to the reference state based on said comparison, e.g. on a direct image comparison (e.g. difference image) and/or corresponding pairs of image features and/or landmarks in the reference and further image/spatial data. The processor may be adapted to provide the output data, in which the output data comprises an alert to the operator when said difference measure exceeds a predetermined threshold, so as to indicate that the subject is no longer in the intended reference position. Additionally or alternatively, a signal may be provided to the diagnostic imaging system so as to disable the execution of the diagnostic image acquisition until the subject is repositioned (or until the operator overrides such disabling signal) if the difference measure exceeds the predetermined threshold.

In a third aspect, the present invention relates to a diagnostic imaging system, in which the system is adapted to perform a method in accordance with embodiments of the first aspect of the present invention and/or comprises a device in accordance with embodiments of the second aspect of the present invention. In a fourth aspect, the present invention relates to a workstation for a diagnostic imaging system, in which the workstation is adapted to perform a method in accordance with embodiments of the first aspect of the present invention and/or comprises a device in accordance with embodiments of the second aspect of the present invention. The diagnostic imaging system may be a diagnostic (e.g. medical or veterinary) X-ray imaging system, e.g. a projection X-ray imaging system (e.g. for planar projection radiography).

For example, Fig. 4 illustrates, schematically, a diagnostic X-ray projection imaging system 30 in accordance with embodiments of the present invention. Such X-ray imaging system typically comprises an X-ray detector 31 for acquiring an X-ray image of a subject 32 by means of modulation of properties of an X-ray beam 33, emitted by an X-ray tube 34, when propagating through the subject. Operation of the system is typically controlled by a workstation 35 (e.g. a control console), e.g. to configure settings of the system, to control (e.g. the activation of) the X-ray tube 34 and/or to acquire an image from the detector 31. The workstation may, for example, also be configured to view the acquired X-ray image. The system comprises a device 10 in accordance with embodiments of the present invention, which may be (optionally) integrated (e.g. at least partially) in the workstation 35, or may be provided as, essentially, a completely isolated system (e.g. for ease of installation, e.g. in an upgrade and/or portable installation). The camera system 11 of the device 10 in accordance with embodiments is configured to monitor the subject 32, e.g. such that at least the relevant anatomy of the subject (in the example shown, the head) for the diagnostic imaging examination to be performed is included in the view cone(s) 37 of the camera(s) of the camera system.

In a fifth aspect, the present invention relates to a computer-program product for performing, when executed on a computer (e.g. a device in accordance with embodiments of the second aspect of the present invention), a method in accordance with the first aspect of the present invention. Other features, or details of features, described hereinabove of a device (resp. computer-program product, system and workstation) in accordance with embodiments of the present invention shall be clear in view of the description provided hereinabove relating to a method in accordance with embodiments of the present invention, and/or vice versa.

## Claims

1. A device (10) for detecting movement of at least one body part of a subject in a diagnostic imaging examination, wherein said device comprises:
- a camera and/or three-dimensional, 3D, surface scanning system (11) for acquiring image and/or spatial data of the subject,
- an input (14) for receiving a trigger signal to indicate that a current spatial configuration of the subject is to be maintained for the diagnostic imaging examination,
- a processor (12) and
- an output (13),
- in which said processor (12) is adapted for:
- acquiring reference image and/or spatial data of the subject using the camera and/or 3D surface scanning system when said trigger signal is received, such that said reference image and/or spatial data represents a reference state of the subject at substantially the time that the trigger signal was received,
- acquiring further image and/or spatial data of the subject using the camera and/or 3D surface scanning system after said reference image and/or spatial data of the subject is acquired,
- comparing the further image and/or spatial data to the reference image and/or spatial data, and
- providing output data via said output (13) to an operator and/or to the subject, wherein said output data is representative of said comparison so as to indicate movement of the subject with respect to the reference state of the subject.

2. The device of claim 1, wherein said processor (12) is adapted to repeat the steps of acquiring the further image and/or spatial data via the camera and/or 3D surface scanning system (11), comparing the further image and/or spatial data to the reference image and/or spatial data, and providing the output data, so as to present a dynamic view of a current change of the spatial configuration of the subject with respect to the reference state of the subject.

3. The device of any of the previous claims, wherein said output (13) comprises a display monitor (15) and said processor (12) is adapted for presenting, via said output data, the shape, area and/or volume of a two-dimensional and/or three-dimensional difference determined by said comparison as an overlay and/or contour on a visualization of the further image and/or spatial data and/or as a marker and/or annotation accompanying said visualization.

4. The device of any of the previous claims, wherein said processor (12) is adapted for determining a difference measure indicative of movement of the subject relative to the reference state based on said comparison, and outputting, in said output data, an alert to the operator and/or the subject and/or a signal to the diagnostic imaging system when said difference measure exceeds a predetermined threshold, so as to indicate that the subject is no longer in the reference state.

5. The device of any of the previous claims, wherein said camera and/or 3D surface scanning system (11) comprises at least one camera and/or 3D scanning device arranged so as to obtain a two-dimensional and/or three-dimensional view of the at least one body part and/or of the subject, wherein said at least one camera and/or 3D scanning device comprises an optical camera for acquiring a monochrome, color and/or multispectral two-dimensional image, in the infrared and/or visible spectrum, and/or a plurality of such cameras configured so as to view the subject from different angles, and/or a depth camera and/or a three-dimensional surface imaging system.

6. The device of any of the previous claims, wherein said input (14) comprises a human interaction interface, a button (17), a voice control interface (18) and/or a gesture detection system (19).

7. The device of any of the previous claims, wherein said input (14) comprises a proximity and/or position detection system (16) to detect proximity of the operator to the subject, so as to generate said trigger signal by taking into account when the operator leaves the vicinity of the subject.

8. The device of claim 7, wherein said proximity and/or position detection system (16) comprises a radiofrequency identification tag in combination with at least one radiofrequency identification tag detection sensor, and/or comprises at least one light gate, and/or comprises a sonar, radar and/or lidar system, and/or wherein said proximity and/or position detection system (16) is implemented, by the processor (12), by detecting and/or tracking the position of the operator in a live stream of image and/or spatial data acquired by the camera and/or 3D surface scanning system (11).

9. The device of any of the previous claims, wherein said input (14) comprises a connection for receiving the trigger signal from an automated system for detecting a predetermined reference spatial configuration of the subject, as indicated for the diagnostic imaging examination, in a live imaging stream provided by the camera and/or 3D surface scanning system (11).

10. The device of any of the previous claims, further comprising an artificial intelligence module for evaluating a trained machine learning model and to generate said trigger signal by taking an output of the evaluated model into account, in which the trained machine learning model uses, as input, said image and/or spatial data and/or data from the diagnostic imaging system, wherein said data from the diagnostic imaging system comprises device state information of the diagnostic imaging system and/or control input information received by the diagnostic imaging system from user interactions.

11. The device of any of the previous claims, wherein said processor is adapted for comparing the further image and/or spatial data with the reference image and/or spatial data by detecting at least one image feature and/or landmark in the reference image and/or spatial data as well as in the further image and/or spatial data, and by comparing a position of the at least one image feature and/or landmark between the reference image and/or spatial data and the further image and/or spatial data.

12. A diagnostic imaging system (30) comprising a device (10) in accordance with any of the previous claims.

13. The diagnostic imaging system of claim 12, wherein said diagnostic imaging system is an X-ray imaging system or a projection X-ray imaging system.

14. A method (100) to detect movement of at least one body part of a subject in a diagnostic imaging examination, the method comprising:
- receiving (101) a trigger signal to indicate that a current spatial configuration of the subject is to be maintained for the diagnostic imaging examination;
- when said trigger is received (101), acquiring (102) reference image and/or spatial data of the subject using a camera and/or 3D surface scanning system;
- after said reference image and/or spatial data of the subject is acquired (102), acquiring (103) a further image and/or spatial data of the subject using the camera and/or 3D surface scanning system;
- comparing (104) the further image and/or spatial data to the reference image and/or spatial data, and
- providing (105) an output to an operator and/or to the subject that is representative of said comparison of the further image and/or spatial data to the reference image and/or spatial data to indicate movement of the subject with respect to the reference state of the subject represented by the reference image and/or spatial data.

15. A computer-program product for performing, when executed on a computer, the method in accordance with claim 14.
